# EUROPEAN PATENT APPLICATION

(11) **EP 2 106 799 A1**
(43) Date of publication of application: **07.10.2009**
(21) Application number: 08006496.7
(22) Date of filing: 31.03.2008
(51) Int. Cl.: A61K 35/16

(54) **Use of a biologically active blood serum for the treatment of a disorder characterized in a reduced function of a GABA receptor**

(71) Applicant: OWEN Holding LTD, Douglas Isle of Man IM 99 3DU (GB)
(72) Inventor: The designation of the inventor has not yet been filed
(74) Representative: Zwicker, Jörk

(57) **Abstract**

The present invention relates to the use of a pharmacologically active blood serum product producible by a method comprising electrostimulation of a non-human animal, withdrawal of blood from said animal, isolation of serum from said blood, and gamma irradiation of said serum for the prevention or treatment of a disorder characterized in a reduced GABA receptor function.

## Description

The present invention relates to the use of a pharmacologically active blood serum product producible by a method comprising electrostimulation of a non-human animal, withdrawal of blood from said animal, isolation of serum from said blood, and gamma irradiation of said serum for the prevention or treatment of a disorder characterized by a reduced GABA receptor function.

### Background of the Invention

GABA (4-aminobutanoic acid) is an endogenous neurotransmitter in the central and peripheral nervous system. In vertebrates, GABA acts at inhibitory synapses in the brain by binding to specific transmembrane receptors in the plasma membrane of both pre- and postsynaptic neurons. Receptors for GABA have historically been divided into GABAA and GABAB receptor subtypes. Today, three classes of GABA receptors are known: GABAA, GABAB, and GABAC. GABAA and GABAC receptors are ligand-gated ion channels (also known as ionotropic receptors), whereas GABAB receptors (for a review see Kerr, D.I.B. and Ong. J. (1995) Pharmac. Ther. vol. 67,pp.187-246) belong to the superfamily of G-protein coupled receptors (also known as metabotropic receptors).

A reduced GABA receptor activity in the central or peripheral nervous system can lead, depending on the genetic predisposition and age of the affected subject, to a variety of disorders including, for example, gut motility disorders such as irritable bowel syndrome and Schizophrenia (see Tamminga CA. , "The neurobiology of cognition in schizophrenia", J. Clin. Psychiatry; 2006 Sep;67(9):e11) but also to several other diseases as will be described in the following.

Substance abuse, such as excessive consumption of alcohol can alter the function of a number of transporters as well as neurotransmitter receptors, including GABA receptors. For example, Santhakumar V et. al. have shown that ethanol acts directly on extrasynaptic subtypes of GABAA receptors to increase tonic inhibition (Alcohol. (2007) May;41(3):211-21.). Thus, GABA receptor agonists can be administered when treating a disorder associated with substance abuse (Nagy J., "Recent patents on pharmacotherapy for alcoholism.", Recent Patents CNS Drug Discov. 2006 Jun;1(2):175-206).

GABA agonists can also counteract age-related disorders because particular subunits of the GABA(A) receptor are altered with age, such that administered agonists help balancing the lost functionality (see Rissman RA, et. al, J Neurochem. 2007 Nov;103(4):1285-92. Epub 2007 Aug 20).

Interestingly, GABAergic neurons are widely distributed throughout the nervous system, including regions of the spinal cord dorsal horn known to be important for transmitting pain related signals to the brain. In addition, supraspinal sites known to coordinate the perception and response to painful stimuli are known to comprise GABA sensitive neurons. In fact, GABA receptors have been shown to regulate and control sensory information processing in the spinal cord. The discovery that GABA receptor agonists display antinociceptive properties in a variety of animal models of pain has provided an impetus for developing compounds which can activate GABA receptors. It has been shown that GABA receptor agonists, as well as inhibitors of GABA uptake or metabolism, are clinically effective in treating pain. However, it has proven difficult to design novel analgesics that can be employed for the routine management of pain. Work in this area has revealed several reasons why GABAergic drugs currently on the market have been of limited utility in the management of pain. Chief among these are the side effects generally associated with such agents. Thus, there is a need for further compounds capable of stimulating GABA receptor function and which are well tolerated *in vivo* (see Enna SJ, et. al., "The role of GABA in the mediation and perception of pain", Adv Pharmacol. 2006; 54:1-27).

It has also been reported that GABA receptor subunits undergo changes in patients suffering from Huntington's disease (e.g., see Thompson-Vest NM, et. al, "GABA(A) receptor subunit and gephyrin protein changes differ in the globus pallidus in Huntington's diseased brain", Brain Res. 2003 Dec 24;994(2):265-70). Furthermore, it is well established that activation of GABA(A) receptors favors sleep (for review, see: Gottesmann C., "GABA mechanisms and sleep", Neuroscience; 2002; 111(2):231-9). Thus, agents which act as GABA receptor agonist can also be used for the therapy of insomnia.

Multiple sclerosis is considered to be an autoimmune disease. In this regard, an individual's immune system can attack the myelin sheath that surrounds nerve cells. This damage leads to muscle weakness, paralysis, poor coordination, balance problems, fatigue, and possible blindness. GABA agonists such as baclofen are known to be effective, when symptomatically treating Multiple sclerosis.

In summary, agents which stimulate GABA receptors are useful medicaments which can be administered for the prophylaxis and treatment of above mentioned diseases. However, due to harmful side effects of many GABA receptor agonists on the market and due to genetic variance in the patients, tolerance to GABA receptor agonists can be limited. Thus, there is still a large need in the art to develop novel therapeutic compounds capable of preventing, treating or ameliorating the severe health consequences of disorders which are characterized by the reduced function of one or more GABA receptors.

### Summary of the Invention

It has surprisingly been found by the present inventor that a biologically active blood serum product producible by a method comprising electrostimulation of a non-human animal, withdrawal of blood from said animal, isolation of serum from said blood, and gamma irradiation of said serum effectively counteracts reduced GABA receptor function. As shown in the examples, the blood serum of the invention is capable of, for example, attenuating discharges in hippocampus slices when they are treated with GABA antagonists such as pentylenetetrazole and bicuculline.

Thus, in one embodiment, the present invention provides the use of a biologically active blood serum producible according to a method comprising the steps of:
a) electrostimulation of a non-human animal
b) withdrawal of blood from said animal,
c) isolation of serum from said blood, and
d) gamma irradiation of said serum
for the manufacture of a medicament for the prevention or therapy of a disorder characterized by a reduced function of a GABA receptor.

In a further aspect the invention concerns a biologically active blood serum producible according to a method comprising the steps of:
a) electrostimulation of a non-human animal
b) withdrawal of blood from said animal,
c) isolation of serum from said blood, and
d) gamma irradiation of said serum
for the prevention or therapy of a disorder characterized by a reduced function of a GABA receptor.

The invention also provides in a further aspect a pharmaceutical composition comprising:
(i) a biologically active blood serum producible according to a method comprising the steps of:
   a) electrostimulation of a non-human animal
   b) withdrawal of blood from said animal,
   c) isolation of serum from said blood, and
   d) gamma irradiation of said serum
   and
(ii) at least one pharmaceutically active ingredient selected from the group consisting of an analgesic, a GABA receptor agonist, an acetylcholinesterase inhibitor, a corticosteroid, an NMDA receptor antagonist, a dopamine receptor blocker, creatine, CoQ10, minocycline, a serotonin reuptake inhibitor (SSRI), a dopamine antagonist, a dopa decarboxylase inhibitor, L-dopa, a catechol-O-methyl transferase inhibitor, a monoamine oxidase-B (MAO-B) inhibitor, an antiglutamatergic agent, CEP 1347, CTCT346, a lazaroid, erythropoietin, methylprednisolone a dopamine receptor and derivatives thereof.

### Detailed Description of the Invention

Methods for the preparation of substances with some pharmacological activity from blood serum are known in the art. One is based on the withdrawal of blood from humans or animals, the subsequent incubation as well as the separation of the active substance and finally the preservation of the substance (see, for example, JP 2123287, EP 0 542 303, RU 2096041, RU 2120301). The prior art method concerned the preparation of a blood serum which improves the resistance of the body in respect of exogenic and endogenic factors like air pressure, air temperature, gravity, light etc. as well as hunger, thirst, sleeping and sexual desires etc. The blood serum is drawn from the donor who has previously been brought into a certain functional state and according to the length of the application of the functional state and the type of functional state, e.g. alcohol abuse, nicotine abuse etc., blood serum with different biological activity can be obtained which shows mitogenic, opthalmogenic, audio active, thermo active, dietary active, sexually active, anti-hypoxic, anti-alcohol and anti-nicotine activity.

A different method is disclosed in EP 1 283 047 and concerns the treatment of animal blood serum by gamma irradiation with the aim to increase the biological activity of the blood serum product.

The present inventor has surprisingly found that a biologically active blood serum isolated from non-human animals upon the stimulation of animals, in particular of chickens with electric currents and further treatment of the serum with γ-radiation can be used for the prophylaxis and treatment of a disorder characterized by a reduced function of a GABA receptor. This was neither mentioned nor suggested in the aforementioned prior art. The biologically active blood serum surprisingly is a an effective agent capable of, for example, attenuating pathological discharges in hippocampus slices which have been treated with GABA receptor antagonists and protecting against low serum cell stress, β-amyloid aggregates and against the cytotoxic effects of elevated L-glutamate concentrations.

Accordingly, in a first aspect the invention provides a use of a biologically active blood serum producible according to a method comprising the steps of:
a) electrostimulation of a non-human animal
b) withdrawal of blood from said animal,
c) isolation of serum from said blood, and
d) gamma irradiation of said serum
for the manufacture of a medicament for the prevention or therapy of a disorder characterized by a reduced function of a GABA receptor.

In a second aspect, the invention provides a biologically active blood serum producible according to a method comprising the steps of:
a) electrostimulation of a non-human animal
b) withdrawal of blood from said animal,
c) isolation of serum from said blood, and
d) gamma irradiation of said serum
for the prevention or therapy of a disorder characterized by a reduced function of a GABA receptor.

The GABA receptor is preferably selected from a GABAA, GABAB and/or GABAC receptor and most preferably the GABA receptor is a GABAA receptor. It is further preferred that the receptor is a mammalian GABA receptor in particular a human GABA receptor.

The loss of GABA receptor function can be routinely determined by the skilled person as described in the examples provided in this specification. Specifically, a brain tissue biopsy of a patient suffering from a disorder characterized by a reduced function of a GABA receptor would exhibit epilepsy-like discharges similar to those that are obtained when a comparable healthy brain tissue sample is brought in contact with pentylenetetrazol and/or bicuculline (see examples herein for a detailed description of the assay). Alternatively, also GABA receptor immunohistochemistry can be used on brain biopsies from patients to detect a differential loss, preferably a loss of more than 5%, 10%, 20%, 30% or more than 50% of a GABA receptor subunit in brain samples of patients when compared to a representative healthy brain tissue sample. Suitable antibodies specific for GABA receptors and GABA receptor subunits are available in the art. The function of a GABA receptor can also be determined without undue burden by measuring the effects of γ-aminobutyric acid (GABA) on the uptake of ³⁶Cl⁻ into a membrane preparation from isolated nerve cords. A review describing such methods has been published by J. Kardos ("The GABAA receptor channel mediated chloride ion translocation through the plasma membrane: New insights from 36Cl- ion flux measurements", Synapse, volume 13, issue 1 , p. 74 - 93). Thus, a patient which is suffering from a disorder caused by a compromised GABA receptor function preferably has GABAA, and/or GABAC receptors, which conduct less than 95%, 90%, 80%, 70%, 60%, 50%, 40%, or less than 30% and more preferably less than 90% of chloride ions through the receptor channel when contacted with GABA or a respective receptor agonist than is conducted by a comparable GABAA or GABAC receptor from a healthy subject (see also: Cash DJ, et. al., "Gamma-Aminobutyric acid (GABA) mediated transmembrane chloride flux with membrane vesicles from rat brain measured by quench flow technique: kinetic homogeneity of ion flux and receptor desensitization"; Life Sci. 1987 Jul 27;41(4):437-45.

Various non-human animals can be used in the production of the biologically active blood serum of the invention, however, it is preferred that the non-human animal is selected from the group consisting of mammals and birds. Because of their easy availability it is particularly preferred to use farm animals like poultry, e.g. chicken, duck, turkey, goose, ostrich and quail. A particular preferred animal which can be used in the method of the present invention is a chicken. The type of mammal that can be used in the method of the present invention is not particularly restricted and comprises without limitation rodents and farm animals, e.g. mice, hamsters, rats, cats, dogs, horses, donkeys, sheep, cows, pigs and goats.

Without wishing to be bound by any theory, it is envisioned by the present inventor that the electrostimulation leads to the release of certain compounds within the animal which cause and/or contribute to the surprising effect of the biological active blood serum of the present invention on GABA receptor function. The non-human animal can be stimulated in different regions of the body. Preferably the electrostimulation is carried out at the head, the neck, the body and/or on one or more of the limbs. It is preferred that the electrostimulation is carried out in a water bath. It is possible to stimulate the body only at one position or at several positions at once. A particular preferred body part for the electrostimulation is the head of the respective animal. When stimulating birds, in particular chicken it is preferred that the head is electrostimulated. In the context of the present invention the terms electrostimulation and electroshock are used interchangeably.

The electrostimulation can be carried out by art known methods, preferably using metal electrodes or water baths as used, for example, during culling of cattle or electrocution of poultry. Most preferably, a water bath is used. Preferably, the electrostimulation is carried out for a time period of between 1 and 60 seconds, preferably between 1 and 30 seconds, more preferably between 2 and 10 seconds, and most preferably at least 4 seconds. The length of the time period of electro stimulation will usually be longer in case that a large animal is electrostimulated and will usually be shorter in cases were small animals are electrostimulated. For example, for the stimulation of chicken heads a particular preferred time period of the electrostimulation is between 2 and 10 seconds and more preferably between 4 and 5 seconds. The other variables which can be adapted during the electrostimulation of the animal is the voltage, the current and the frequency of the current and the present inventors have defined certain preferred ranges for these parameters. The actual parameter chosen will depend in part on the size of the animal as well as on the region of the animal to be stimulated. In general, larger animals and larger regions will require a higher voltage and current. Thus, the electro stimulation is preferably carried out with a voltage in the range of between 50 Volt and 150 Volt, preferably 80 Volt to 120 Volt and more preferably between 110 Volt and 120 Volt. The ranges for the currents that can be applied are between 0.01 A and 1.0 A, preferably between 0.02 A and 1.0 A, more preferably between 0.04 A and 0.1 A and most preferably at least 0.06 A. Voltage, current and application time are preferably chosen to administer energy in the range of between 1 and 1,000 Ws, preferably in the range of 10 to 200 Ws and even more preferably in the range of 15 to 100 Ws.

For the stimulation of the preferred animals, i.e. chicken, it is preferred that the electrostimulation is carried out with a voltage in the range of between 80 Volt to 120 Volt and more preferably between 110 Volt and 120 Volt. Furthermore, a current in the range of between 0.04 A and 0.08 A, in particular of at least 0.06 A is preferred in the context of the electrostimulation of birds, in particular of chicken. Preferably, the electrostimulation of a bird, in particular a chicken, is carried out for between 3 and 60 seconds, preferably for at least 4 seconds at a voltage of between 80 V and 120 V, in particular between 110 V and 120 V. In this preferred embodiment the current is preferably between 0.04 A and 0.1 A and most preferably at least 0.06 A.

The frequency of the electrostimulation does not appear to be particularly critical but is preferably in the range of between 10 and 200 Hertz, more preferably in the range of between 50 to 100 Hz and most preferably around 50-60 Hz.

Preferably, if the electrostimulation acts as an anaesthesia, the time period between electrostimulation and exsanguinations is smaller than 20 seconds more preferably smaller than 10 seconds. With respect to the above mentioned electrostimulation parameters it is preferred that they are selected such that they are permissible under the respective federal animal protection regulation (such as, e.g. TierSchlV in Germany) of the respective country at which the electrostimulation is carried out.

The gamma irradiation of the serum during step d) of the method of producing the biologically active blood serum can be carried out with any gamma source including X-ray sources and radionuclides. Preferably the gamma irradiation source is a radionuclide with a defined gamma irradiation pattern. Preferred sources for the gamma irradiation are selected from the group consisting of ⁶⁰Co, ¹³⁷Cs, ⁶⁷Cu, ⁶⁷Ca, ¹¹¹In, ¹⁹²Ir, ^{99m}Tc and ¹⁷⁰Tm. Out of those ⁶⁰Co, ¹³⁷Cs, ¹⁹²Ir and ¹⁷⁰Tm are particular preferred with ⁶⁰Co being the most preferred gamma radiation source for use in the method of the present invention.

The radiation dose adsorbed by the serum is preferably in the range of between 10 to 40 kGy preferably in the range of between 15 to 35 kGy and more preferably in the range of between 20 and 30 kGy, i.e. 25 ± 5 kGy. Gamma irradiation on one hand sterilizes the serum and on the other bolsters the activity of the serum. The suitable irradiation time can vary, depending on the desired level of activity of the resulting serum and the respective indication for which the serum is to be used. The experimental section describes assays to test the activity of the serum of the invention and these tests can be used by someone of skill in the art to determine the optimal duration of the application of irradiation without undue burden. Preferably the irradiation is carried out for a period of time in the range of 30 min to 10 hours, preferably for 45 min to 8 h and more preferably for 1 h to 5 h, i.e. for 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295 or 300 min. It is further preferred that the energy dose applied for this period of time is within a range of 10 to 40 kGy, preferably 15 to 35 kGy and more preferably between about 20 to 30 kGy. Again the preferred radiation source in this context is ⁶⁰Co. Thus, it is particularly preferred that irradiation is carried out in the range of 30 min to 10 hours, preferably for 45 min to 8 h and more preferably for 1 h to 5 h, i.e. for 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295 or 300 min. at an energy dose between about 20 to 30 kGy.

The withdrawal of the blood from the animal can be affected by any art known method and includes syringes as well as puncturing of arteries or veins or decapitation in particular in the context of the withdrawal of blood from birds. It is possible to withdraw only a part of the blood or to completely withdraw the blood of the animal. The later is preferably used, if a lethal dose of electricity has been applied to the animal. The withdrawn blood can be arterial and/or venous blood.

The serum can be isolated from the blood by any art known method including filtration, sedimentation and centrifugation. It is, however, preferred that the blood is incubated for between 4 and 72 h at a low temperature, e.g. between 2°C and 10°C, preferably between 4°C and 8°C to allow clotting of the blood which leads to the release of additional factors into the blood. Thus, it is preferred that the method of producing the biologically active blood serum further comprises the step of incubating the blood after the withdrawal of the blood from the animal and prior to the isolation of the serum from the blood, e.g. for between 4 and 72 h at a low temperature, e.g. between 2° and 10°C, preferably between 4°C and 8°C.

In a further preferred embodiment of the use of the present invention the method for producing the biologically active blood serum comprises the further step of lyophilization of the serum prior to the irradiation step d). The lyophilization allows easier handling of the serum during irradiation and optimizes absorption of the radiation by the serum components.

In one preferred embodiment, the biologically active blood serum used in the various embodiments of the invention is producible according to the method described in example 1.

As mentioned above, it was an unexpected finding of the inventor that the biologically active blood serum according to the invention counteracts, as shown in the examples, the effects of reduced GABA receptor activity. It was further demonstrated that mammalian nerve cells, e.g. pyramidal cells and/or primary chicken telencephalon neurons are treatable with the biologically active blood serum of the invention. It has also been shown in the examples that the blood serum according to the invention protects neurons from the cytotoxicity of L-Glutamate. Thus, the blood serum of the invention shows the unexpected synergistic feature of protecting neurons from L-glutamate and also ameliorating the functionality of GABA receptor function. L-Glutamate serves as the precursor for the synthesis of the neurotransmitter GABA. This reaction is catalyzed by glutamic acid decarboxylase (GAD). The movement disorder Stiff-man syndrome is a disorder caused by anti-GAD antibodies, leading to a decrease in GABA synthesis and an increase in L-Glutamate levels. As GABA receptors are also functioning at neuromuscular junctions, this results in impaired motor function such as muscle stiffness and spasm. Thus, the blood serum of the present invention will be particularly effective when used for the prevention or therapy of a movement disorder. In a preferred embodiment the biologically active blood serum of the invention is used for the treatment of a movement disorder selected from the group consisting of Stiff-man syndrome, a gut motility disorder, spasticity and cerebral palsy.

Additionally, as was mentioned above in the introduction, a loss in GABA receptor function can lead to various other diseases, which, thus, are also treatable with the blood serum according to the invention.

Thus, the disorder characterized in a reduced function of a GABA receptor treatable with the biologically active blood serum of the invention is preferably selected from a group consisting of a substance-abuse related disorder, multiple sclerosis, an aging related neuropathy, pain, schizophrenia, Huntington's disease, insomnia, asthma and a movement disorder. In another aspect, the invention provides a biologically active blood serum of the invention for the prevention or therapy of one of the above mentioned specific disorders.

The substance abuse-related disorders may, e.g., arise from the abuse of alcohol, amphetamines (or amphetamine-like substances) caffeine, cannabis, cocaine, hallucinogens, inhalants and aerosol propellants, nicotine, opioids, phenylglycidine derivatives, sedatives, hypnotics, and anxiolytics, while such disorders preferably include dependence and abuse, intoxication, withdrawal, intoxication, delirium and withdrawal delirium.

Examples of the types of pain that can be treated with the serum of the present invention and their pharmaceutical acceptable salts include pain resulting from soft tissue and peripheral damage, such as acute trauma, pain associated with osteoarthritis and rheumatoid arthritis, musculo-skeletal pain, such as pain experienced after trauma; spinal pain, dental pain, myofascial pain syndromes, episiotomy pain, and pain resulting from bums; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, trigeminal neuralgia, neuropathic lower back pain, HIV related neuropathic pain, cancer related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting fromankylosing spondylitis and gout; pain caused by increased blader contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain.

As the biologically active blood serum of the invention can be used to prevent or treat a disorder characterized by a reduced function of a GABA receptor, such as a substance-abuse related disorder, multiple sclerosis, an aging related neuropathy, pain, schizophrenia, Huntington's disease, insomnia, asthma and a movement disorder, it can, in another aspect of the invention, also be admixed to a pharmaceutically active ingredient which is known in the art to be effective for the treatment or prevention of one of the aforementioned disorders.

Thus, the invention also provides in a further aspect a pharmaceutical composition comprising:
(ii) a biologically active blood serum producible according to a method comprising the steps of:
   a) electrostimulation of a non-human animal
   b) withdrawal of blood from said animal,
   c) isolation of serum from said blood, and
   d) gamma irradiation of said serum
   and
(ii) at least one pharmaceutically active ingredient selected from the group consisting of an analgesic, a GABA receptor agonist, an acetylcholinesterase inhibitor, a corticosteroid, an NMDA receptor antagonist, a dopamine receptor blocker, creatine, CoQ10, minocycline, a serotonin reuptake inhibitor (SSRI), a dopamine antagonist, a dopa decarboxylase inhibitor, L-dopa, a catechol-O-methyl transferase inhibitor, a monoamine oxidase-B (MAO-B) inhibitor, an antiglutamatergic agent, CEP 1347, CTCT346, a lazaroid, erythropoietin, methylprednisolone a dopamine receptor and derivatives thereof.

As used herein, the term "derivative" refers to a chemical compound or molecule made from a parent compound or molecule by one or more chemical reactions. Preferably, however, the parent molecule must still be comprised in its structure in a "derivative". Additionally, the derivative preferably has a molecular weight which is not greater than twice the molecular weight of the parent compound or parent monomer, if the compound is comprised in a polymer.

In a preferred embodiment, the acetylcholinesterase inhibitor of the pharmaceutical composition according to the invention is selected from the group consisting of metrifonate, carbamate, physostigmine, neostigmine, pyridostigmine, ambenonium, demarcarium, rivastigmine, a phenanthrine derivative, galantamine, a piperidine, donepezil, tacrine, edrophonium and phenothiazine.

The NMDA receptor antagonist of the pharmaceutical composition is preferably selected from the group consisting of amantadine, dextromethorphan, dextrorphan, ibogaine, ketamine , nitrous oxide, phencyclidine, riluzole, tiletamine, ethanol , dizocilpine, aptiganel, memantine, remacimide, 7-chlorokynurenate, 5,7-dichlorokynurenic, kynurenic acid, 2-amino-7-phosphonoheptanoic acid, r-2-amino-5-phosphonopentanoate and 3-[(R)-2-carboxypiperazin-4-yl]-prop-2-enyl-1-phosphonic acid).

In a preferred embodiment, the dopamine antagonist is selected from the group consisting of clozapine, risperidone, olanzapine, quetiapine, ziprasidone, aripiprazole, metoclopramide, droperidol, domperidone, chlorpromazine and amoxapine.

It is also preferred that the GABA receptor agonist of the pharmaceutical composition according to the invention is selected from the group consisting of β-carbolin-3-carboxamide, FG-7142, etomidat, propofol, gamma-hydroxybutyric acid, benzodiazepine, zolpidem, zopiclone, zaleplon, methaqualone, baclofen, muscimol, progabide, tiagabine, 4-aminobutanoic acid (GABA); 3-aminopropyl)methylphosphinic acid; 4-amino-3-phenylbutanoic acid; 4-amino-3-hydroxybutanoic acid; 4-amino-3-(4-chlorophenyl)-3-hydroxyphenylbutanoic acid; 4-amino-3-(thien-2-yl)butanoic acid; 4-amino-3-(5-chlorothien-2-yl)butanoic acid; 4-amino-3-(5-bromothien-2-yl)butanoic acid; 4-amino-3-(5-methylthien-2-yl)butanoic acid; 4-amino-3-(2-imidazolyl)butanoic acid; 4-guanidino-3-(4-chlorophenyl)butanoic acid; 3-amino-2-(4-chlorophenyl)-1-nitropropane; (3-aminopropyl)phosphonous acid; (4-aminobut-2-yl)phosphonous acid; (3-amino-2-methylpropyl)phosphonous acid; (3-aminobutyl)phosphonous acid; (3-amino-2-(4-chlorophenyl)propyl)phosphonous acid; (3-amino-2-(4-chlorophenyl)-2-hydroxypropyl)phosphonous acid; (3-amino-2-(4-fluorophenyl)propyl)phosphonous acid; (3-amino-2-phenylpropyl)phosphonous acid; (3-amino-2-hydroxypropyl)phosphonous acid; (E)-(3-aminopropen-1-yl)phosphonous acid; (3-amino-2-cyclohexylpropyl)phosphonous acid; (3-amino-2-benzylpropyl)phosphonous acid; [3-amino-2-(4-methylphenyl)propyl]phosphonous acid; [3-amino-2-(4-trifluoromethylphenyl)propyl]phosphonous acid; [3-amino-2-(4-methoxyphenyl)propyl]phosphonous acid; [3-amino-2-(4-chlorophenyl)-2-hydroxypropyl]phosphonous acid; (3-amino propyl)methylphosphinic acid; (3-amino-2-hydroxypropyl)methylphosphinic acid; (3-aminopropyl)(difluoromethyl)phosphinic acid; (4-aminobut-2-yl)methylphosphinic acid; (3-amino-1-hydroxypropyl)methylphosphinic acid; (3-amino-2-hydroxypropyl)(difluoromethyl)phosphinic acid; (E)-(3-aminopropen-1-yl)methylphosphinic acid; (3-amino-2-oxo-propyl)methyl phosphinic acid; (3-aminopropyl)hydroxymethylphosphinic acid; (5-aminopent-3-yl)methylphosphinic acid; (4-amino-1,1,1-trifluorobut-2-yl)methylphosphinic acid; (3-amino-2-(4-chlorophenyl)propyl)sulfinic acid; 3-aminopropylsulfinic acid and 1-(aminomethyl)cyclohexaneacetic acid.

In a further embodiment of the pharmaceutical composition according to the invention, the analgesic is selected from the group consisting of acetaminophen (paracetamol), aspirin, opioid analgesics, such as morphine, codeine, dihydrocodeine, diacetylmorphine, bydrocodone, hydromorphone, levorphanol, oxymorphone, alfentanil, buprenorphine, butorphanol, fentanyl, sufentanyl, meperidine, methadone, nalbuphine, propoxyphene, pentazocine and pharmaceutically acceptable salts thereof.

The biologically active blood serum and the pharmaceutical composition according to the invention can be administered by various well known routes, including oral, rectal, intragastrical, intracranial and parenteral administration, e.g. intravenous, intramuscular, intranasal, intradermal, subcutaneous, and similar administration routes. Parenteral administration and particular intravenous administration, preferably by depot injection, is preferred. Depending on the route of administration different pharmaceutical formulations are required and some of those may require that protective coatings are applied to the drug formulation to prevent degradation of the biologically active serum in, for example, the digestive tract.

Thus, preferably the biologically active blood serum and the pharmaceutical composition according to the invention is formulated as a syrup, an infusion or injection solution, a tablet, a capsule, a capslet, lozenge, a liposome, a suppository, a plaster, a band-aid, a retard capsule, a powder, or a slow release formulation. Preferably the diluent is water, a buffer, a buffered salt solution or a salt solution and the carrier preferably is selected from the group consisting of cocoa butter and vitebesole.

Particular preferred pharmaceutical forms for the administration of the biologically active blood serum or the pharmaceutical composition according to the invention during the use of the present invention are forms suitable for injectionable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. The biologically active blood serum and the pharmaceutical composition used in the present invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti-bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing the biologically active blood serum or the pharmaceutical composition according to the invention is accomplished by incorporating the biologically active serum in the required amount in the appropriate solvent with various ingredients enumerated above as required followed by sterilization. To obtain a sterile powder the above solutions are vacuum-dried or freeze-dried as necessary. Preferred diluents of the present invention are water, physiological acceptable buffers, physiological acceptable buffer salt solutions or salt solutions. Preferred carriers are cocoa butter and vitebesole. Excipients which can be used with the various pharmaceutical forms of the biologically active blood serum can be chosen from the following non-limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

Other suitable excipients can be found in the Handbook of Pharmaceutical Excipients, published by the American Pharmaceutical Association, which is herein incorporated by reference.

Based on the results in animals certain amounts of the biologically active blood serum and the pharmaceutical composition according to the invention are preferred for the prevention or treatment of a disorder characterized by a reduced function of a GABA receptor. It is, however, understood that depending on the severity of the disorder and the particular type, e.g. a substance-abuse related disorder, multiple sclerosis, an aging related neuropathy, pain, schizophrenia, Huntington's disease, insomnia, asthma or a movement disorder, as well as on the respective patient to be treated, e.g. the general health status of the patient, etc., different doses of the biologically active blood serum or the pharmaceutical composition are required to elicit a therapeutic or prophylactic effect. The determination of the appropriate dose lies within the discretion of the attending physician. It is contemplated that the dosage of the biologically active blood serum in the therapeutic or prophylactic use of the invention should be in the range of about 0.1 mg to about 1 g serum per kg body weight. However, in a preferred use of the present invention the biologically active blood serum and/or the pharmaceutical composition according to the invention is administered to a subject in need thereof in an amount ranging from 1.0 to 500 mg/kg body weight, preferably ranging from 10 to 200 mg/kg body weight, preferably ranging from 50 to 150 mg/kg body weight, preferably ranging from 90 to 100 mg/kg body weight. The duration of therapy with biologically active blood serum and/or the pharmaceutical composition according to the invention will vary, depending on the severity of the disease being treated and the condition and idiosyncratic response of each individual patient.

As is known in the art, the pharmaceutically effective amount of a given composition will also depend on the administration route. In general the required amount will be higher, if the administration is through the gastrointestinal tract; e.g. by suppository, rectal, or by an intragastric probe, and lower if the route of administration is parenteral, e.g. intravenous. Typically, the biologically active blood serum and/or the pharmaceutical composition according to the invention will be administered in ranges of 50 mg to 1 g/kg body weight, preferably 100 mg to 500 mg/kg body weight, if rectal or intragastric administration is used and in ranges of 10 to 100 mg/kg body weight, if parenteral administration is used.

If a person is know to be at risk of developing a disorder characterized by a reduced function of a GABA receptor, a prophylactic administration of the biologically active blood serum or the pharmaceutical composition according to the invention is possible. In these cases the biologically active blood serum and/or the pharmaceutical composition according to the invention is preferably administered in above outlined preferred and particular preferred doses on a daily basis. Preferably, between 0.1 mg to 1 g/kg body weight once a day, preferably 10 to 200 mg/kg body weight. This administration can be continued until the risk of developing a disorder characterized by a reduced function of a GABA receptor has lessened. In most instances, however, the biologically active blood serum and/or the pharmaceutical composition according to the invention will be administered once a disorder characterized by a reduced function of a GABA receptor has been diagnosed. In these cases it is preferred that a first dose of the biologically active blood serum and/or the pharmaceutical composition according to the invention is administered one, two, three or four times daily. Preferably the administration is discontinued for one day, one week or one month and then repeated until the symptoms of the respective disease are no longer worsening or are improving.

The following examples are included to demonstrate preferred embodiments of the invention. As used throughout the figures, tables and examples, the term "serum S1" refers to the biologically active blood serum according to the invention. It should be appreciated by those of skill in the art that the techniques disclosed in the examples that follow represent techniques discovered by the inventors to function well in the practice of the invention, and thus, can be considered preferred modes for its practise. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Brief Description of the Figures and Drawings

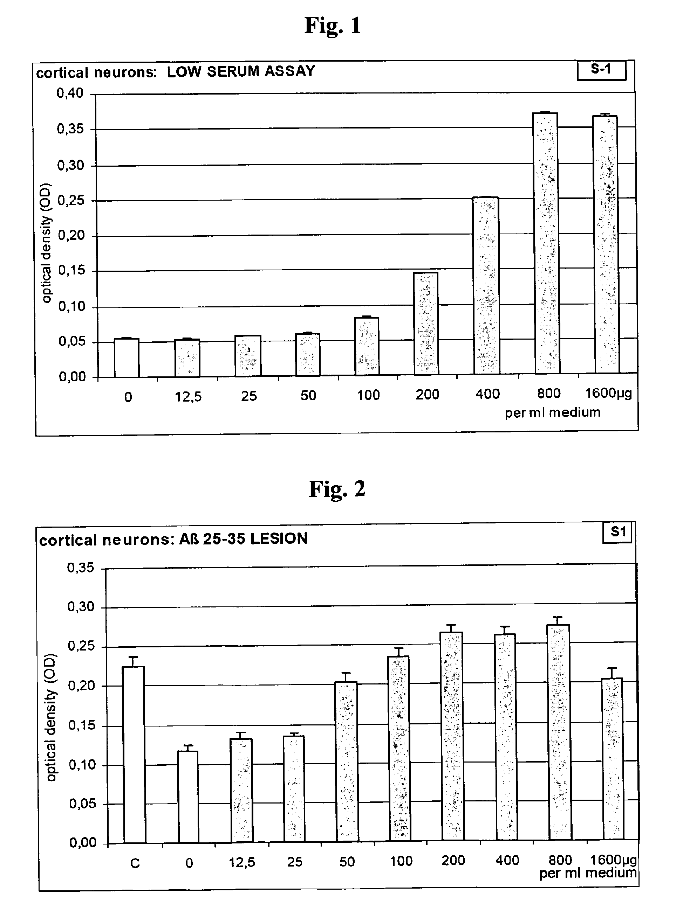

- **Figure 1:**: Effects of serum S1 on neuronal viability obtained in a 2% low serum assay *in vitro*. Neuronal viability as a measure for living cells was obtained with the MTT-test (see example 4 for details on the MTT-test). Values represent the mean ODs ± s.e.m. from 2 independent experiments performed on 2 days with two 96-well plates and 2 wells/concentration (n≤8). Serum S1 was added on the first day. Controls ("0" µg; n≥12) did not receive serum S1.
- **Figure 2:**: Effects of serum S1 on neuronal viability obtained in a β-amyloid₂₅₋₃₅ lesion assay *in vitro.* Neuronal viability as a measure for living cells was obtained with the MTT-test. Values represent the mean ODs ± s.e.m. from 2 independent experiments performed on 2 days with two 96-well plates and 2 wells/concentration (n≤8). Serum S1 was added on the first day. Unlesioned controls ("C"; n≥12) and lesioned controls ("0" µg; n≥12) did not receive serum S1. Pre-aggregated Aβ₂₅₋₃₅ was added on day 8 to a final concentration of 20 µM.

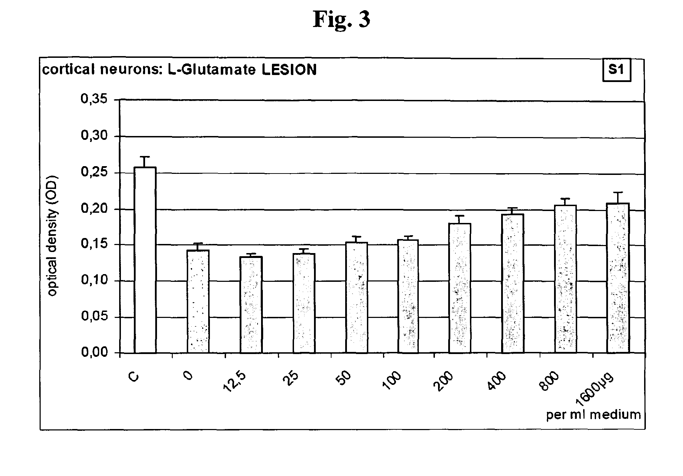

- **Figure 3:**: Effects of serum S1 on neuronal viability obtained in an L-glutamate lesion assay *in vitro.* Neuronal viability as a measure for living cells was obtained with the MTT-test. Values represent the mean ODs ± s.e.m. from 2 independent experiments performed on 2 days with two 96-well plates and 2 wells/concentration (n≤8). Serum S1 was added on the first day. Unlesioned controls ("C"; n≥16) and lesioned controls ("0" µg; n≥16) did not receive serum S1. L-glutamate was added on day 8 to a final concentration of 1mM.

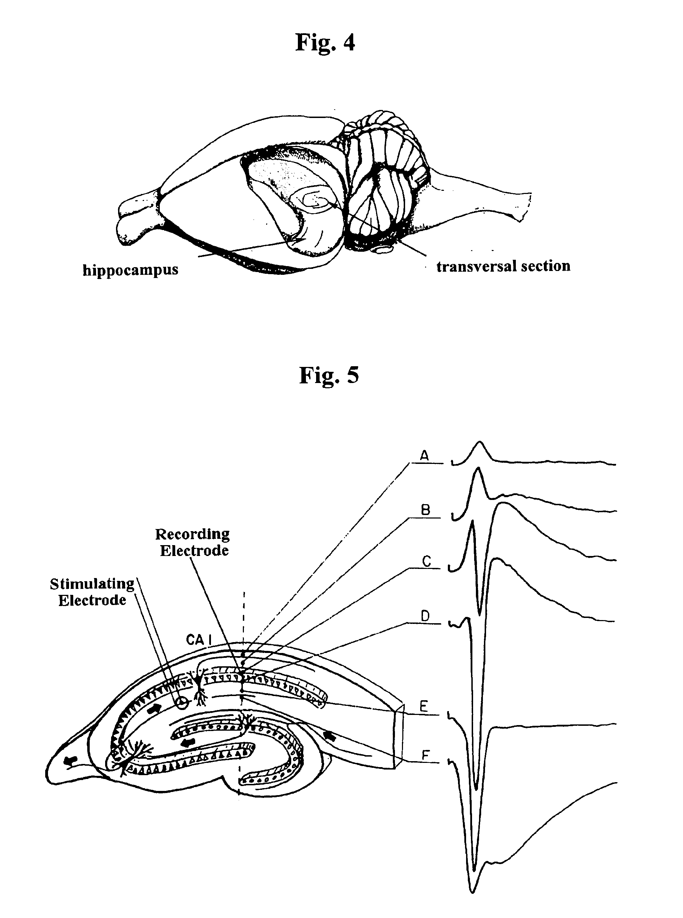

- **Figure 4**: Anatomical view of the position of the hippocampus in the rat. Direction of the cut of slices is depicted (transversal slices are shown in Figure 5).
- **Figure 5**: Documentation of signals as obtained from different recording places of transversal section (Figure 4). In the present study recording was performed from the location "C".

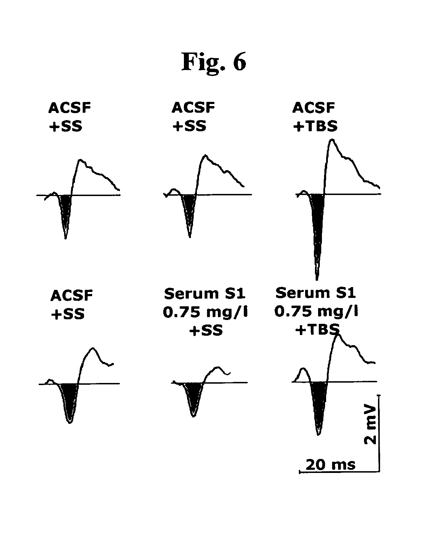

- **Figure 6**: Documentation of representative original signals showing the effect of using single stimuli (SS) or theta burst stimulation (TBS) in the presence of artificial cerebrospinal fluid (ASCF) or serum S1 (0.75 mg/l) diluted in ACSF. The amplitude is calculated from baseline to the down reflection of the signal (shadowed). Scales: Time is given in milliseconds (ms), amplitude of population spike in millivolt (mV).

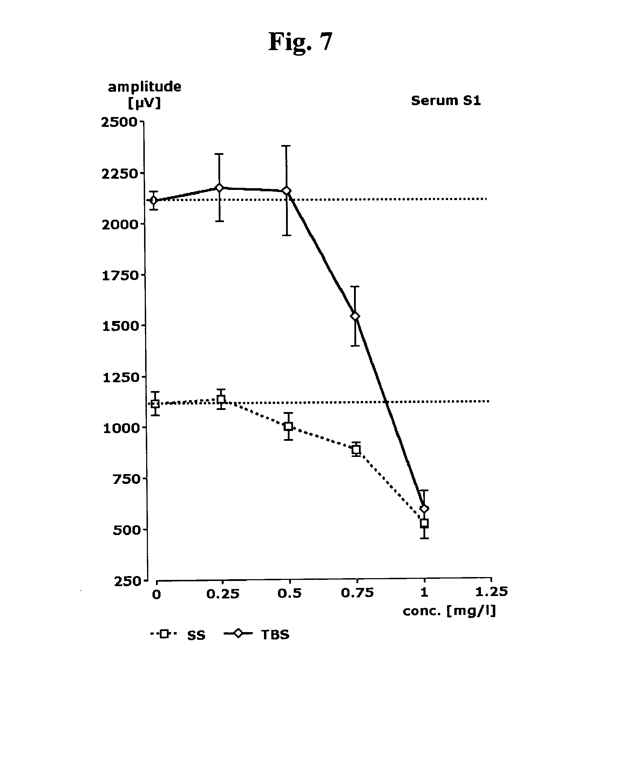

- **Figure 7**: Concentration dependence of amplitude decrease of population spikes in the presence of serum S1 following single stimuli (SS) as well as theta burst stimulation (TBS). Data are presented as mean of n=4 slices ± SEM.

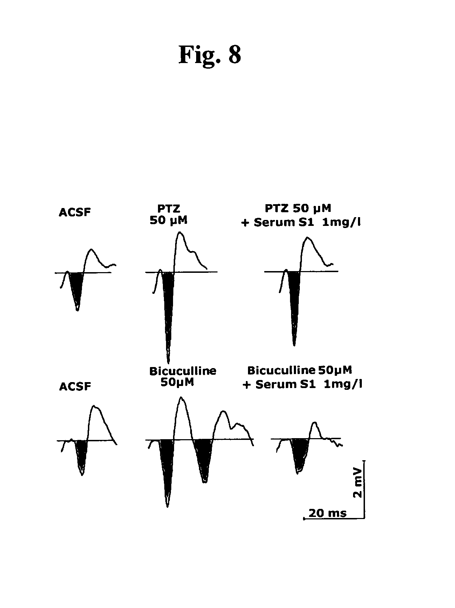

- **Figure 8**: Documentation of original signals showing the effects of pentylenetetrazol (PTZ), bicuculline and in combination of serum S1.

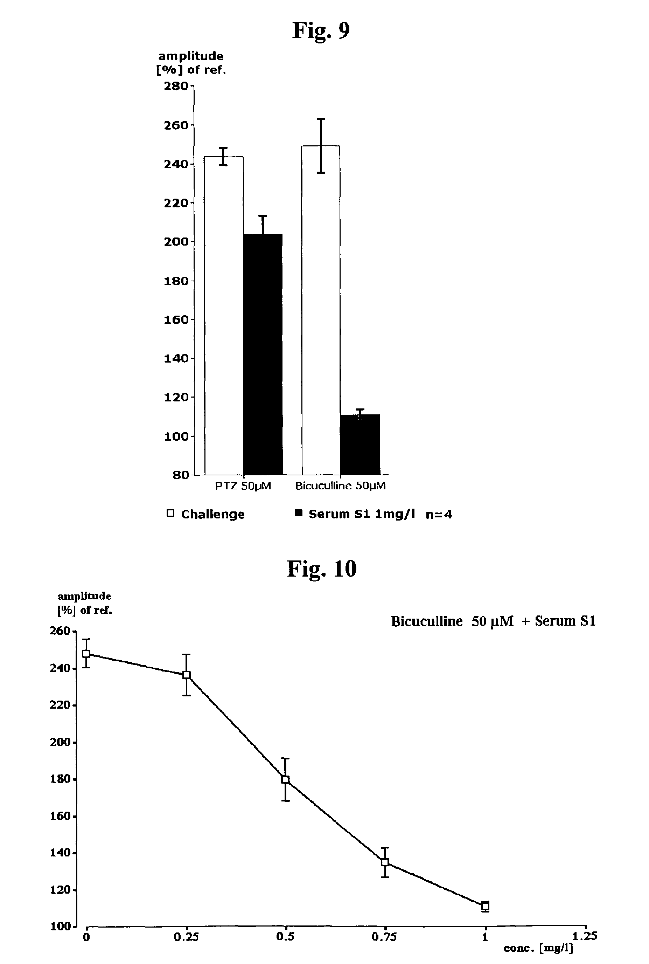

- **Figure 9**: Changes of the population spike amplitude in the presence of two different convulsive drugs. Data are presented for the mean of n=4 slices +- SEM.
- **Figure 10**: Documentation of concentration dependent effects of serum S1 against increases of population spike amplitude induced by bicuculline 50 µM. Scales: serum S1 concentration is given in mg/l on the X-axis, amplitude in % of reference signal (control measurement is before exposure to bicuculline and serum S1). Data are presented for the mean of n=4 slices ± SEM.

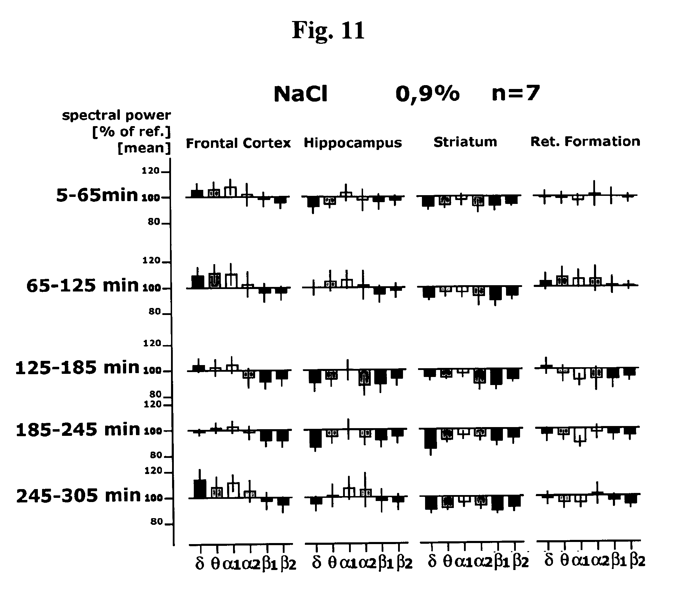

- **Figure 11**: Effects of saline on an electropharmacogram (see examples). Time dependence of changes of spectral power in % of the 45 min lasting pre-drug baseline recording in four brain regions of the freely moving rat. Frequency ranges are depicted as bar graphs representing from left to right delta, theta, alphal, alpha2, beta1 and beta2 spectral power. Data are given as mean +- S.E.M.

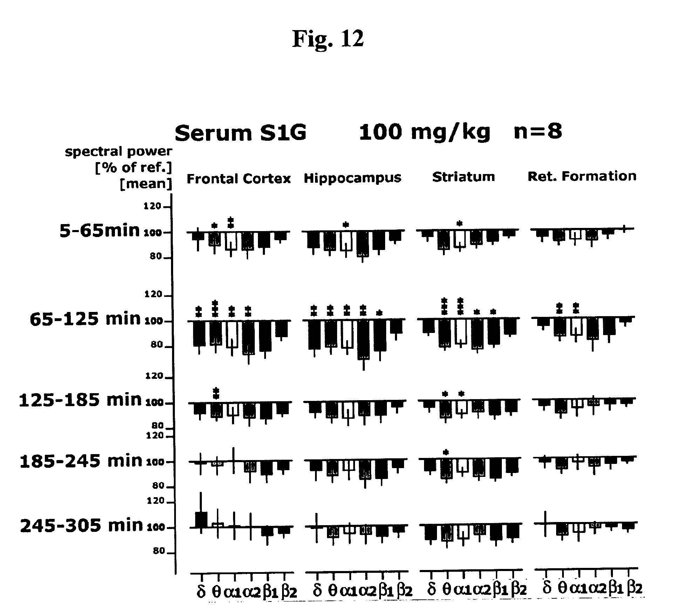

- **Figure 12**: Effects of serum S1 (100.0 mg/kg i.p.) on an electropharmacogram (see examples). For other details see legend to Fig. 11. Statistical significance in comparison to control (vehicle injection) is documented by stars reflecting the error probability: *=p<0.10; **=p<0.05; ***=p<0.01.

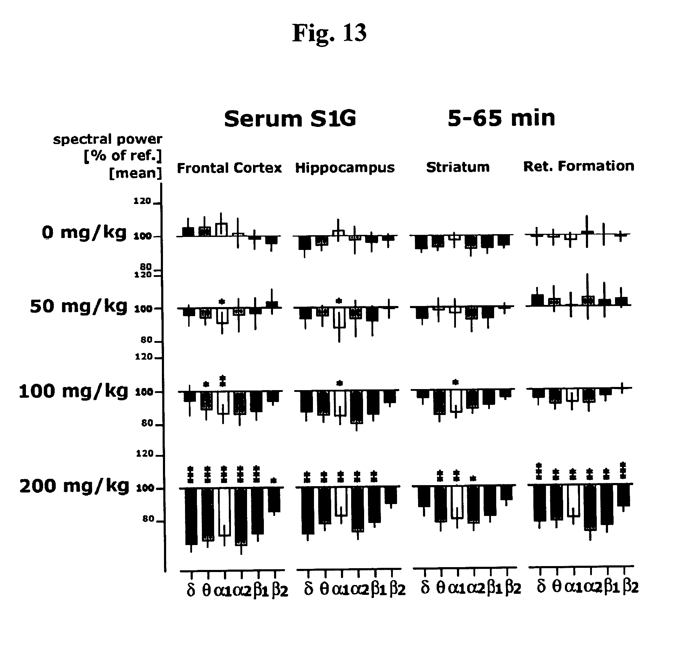

- **Figure 13**: Effects of serum S1 on an electropharmacogram (see examples). Dose dependence of changes of spectral power in % of the 45 min lasting pre-drug baseline recording in four brain regions of the freely moving rat. Frequency ranges are depicted as bar graphs representing from left to right: delta, theta, alpha1, alpha2, beta1 and beta2 spectral power. Statistical significance in comparison to control is given as error probability: *=p<0.10; **=p<0.05; ***=p<0.01.

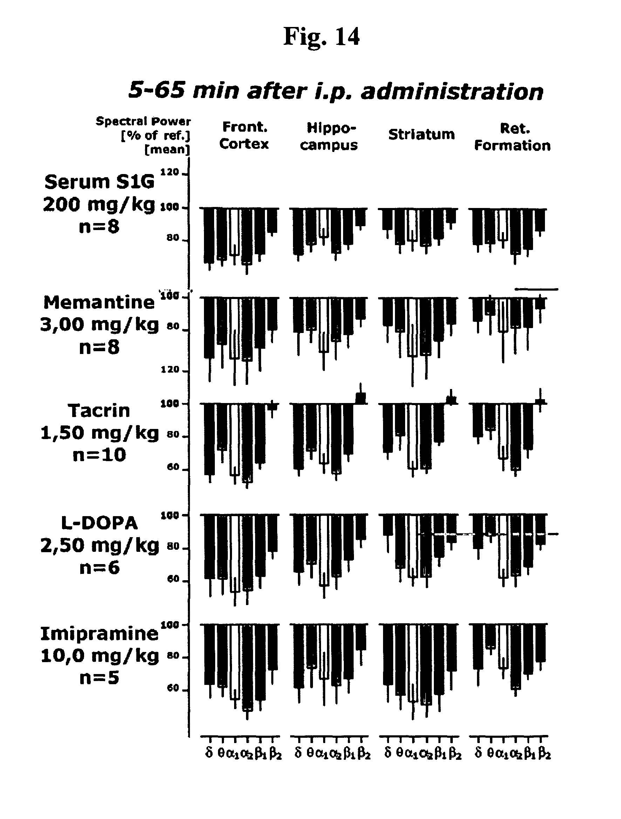

- **Figure 14**: Comparison of the serum S1 with several reference drugs in an electropharmacogram (see examples) during the first hour after administration. The dosage and number of animals tested is given under the name of the drug. Reference drugs are selected on the total pattern of changes in all four brain areas by the relative proportions of frequency changes produced by them.

### Examples

### Example 1. Method of obtaining serum S1 from chicken treated with electroshock

For the preparation of serum S1 from chicken, the chicken were treated with an electroshock of grade II to III (electrical voltage 80-120 V, current 0.06 A, frequency 50 Hz, application time: 4 to 5 sec at the head) in a standard water bath conforming to the standards set out in TierSchlV of Germany. Blood was then drawn from the arteria carotis and further incubated at a temperature of 4°C to 8°C for 18 h to 24 h in polyethylene flasks. After complete retraction of blood clots the flasks were spun at 3.000 rpm for between 20-30 minutes. The serum was separated from the blood clots and lyophilized under art known conditions. The flasks with the lyophilized serum were treated on a RZ-100-M apparatus with 20-30 kGy, preferably at around 25 kGy using ⁶⁰Co as a gamma radiation source for 3 to 4 h. Typically the dried serum is placed into a paper bag of a size of 70 x 75 x 200 mm during irradiation. Radiation strength is adapted to provide about 4.5 kGy/h in the middle of the bag. The treated serum was stored at a temperature of between 4°C to 8°C for later use.

### Example 2. Experiments using chicken neurons

The first study was carried out to assess the neuroprotective effects of serum S1, a chicken blood derivative, using isolated cortical neurons from chick embryos. The effects of the substance was investigated in a 2% low serum cell stress assay, in a β-amyloid lesion assay, as well as in an acute L-glutamate lesion model. These three lesion assays mimic pathologic situations as could also result from a disorder characterized in a reduced function of a GABA receptor.

### Example 3. Cell culture conditions

### 3.1. General Preparations:

All experiments were carried out under sterile conditions, which means all testing was performed in a cell culture unit with special equipment. All items were sterilised prior to the experiments. Stock solutions were purchased already sterile and final solutions were mixed in the laminar airflow cabinet.

### 3.2. Culture Medium:

The medium for the low serum cell stress assay consisted of Eagle's minimal essential medium (EMEM) with 1 g glucose/l, 2% FCS, 0.01% gentamycin and 2 mM L-glutamine. The medium for the β-amyloid and L-glutamate lesion assays consisted of Dulbecco's modified Eagle's medium (DMEM) with 4.5 g glucose/l, 5% Nu serum, 0.01% gentamycin and 2 mM L-glutamine (also referred to herein as "nutrition medium"). L-glutamine is required for growth and differentiation, and gentamycin was added to prevent cell cultures from an infection with mycoplasm or other unwanted microorganism. For each experiment the nutrition medium was freshly prepared in the laminar air flow cabinet under sterile conditions.

### 3.3. Cell Source:

Lohman Brown chicken embryo hybrids were used. One-day-old fertilised eggs are purchased from a local chicken breeder (Schlierbach Geflügel GmbH, Austria) and stored in the lab under appropriate conditions (12 ± 0.3°C and 80 ± 5% humidity). At embryonic day 0 eggs were transferred into a breeding incubator and stored under permanent turning until embryonic day 8 or 9 at 38 ± 0.5°C and 55 ± 5% humidity. For isolation of neurons 3 to 4 chicken embryos were used per experiment. The age of the embryos is very critical, since only in a particular period of development the brain almost exclusively contains nerve cells and less than 5% glia (Pettmann, B., Louis, J.C. and Sensenbrenner, L.M. (1979) Nature 281:378-380).

### 3.4. Preparation and Dissociation:

Eggs were wiped with 70% ethanol and cracked with large forceps at the blunt end. After decapitation of the embryo, the tissue covering the telencephalon or the mesencephalon (depending on the lesion assay performed) was removed. After removing any loose tissue and remaining meningeal membranes, telencephalon brain tissue was transferred into a dish containing nutrition medium. Thereafter, to obtain primary chicken telencephalon neurons, which were used in the low serum cell stress assay, the β-amyloid lesion assay and the L-glutamate lesion assay, the tissue was dissociated mechanically by using a 1 ml pipette and by squeezing 3 times through a sterile nylon sieve with a pore size of 100 µm.

### 3.5. Cell Counting and Determination of Viability of isolated primary neurons:

Using a standard trypan blue dye exclusion test (Sigma), the number of cells and the cell viability was determined after preparation of the primary neuron culture. For cell counting, one part of the cell suspension has to be diluted with 9 parts of trypan blue solution (270 µl PBS and 180 µl 0.5% trypan blue solution). Living cells and blue stained death cells are counted in a Bürker-Türk-hemocytometer. The total number of cells minus the stained dead ones gives the amount of vital cells.

### 3.6. Plating out and Culturing of Nerve Cells:

In the experiments described, poly-D-lysin coated 96-well microtiter plates (Biocoat) were used. 80 µl medium containing 6 x 10⁵ cells /ml were added to each well of the previously prepared microtiter plates already containing 80 µl nutrition medium supplemented with serum S1 to the indicated final concentrations, or without supplement (controls designated "C" and "0" in figures 1, 2 and 3). The final amount of cells in each well is 3 x 10⁵ /ml nutrition medium. When preparing plates, routinely outside wells are filled with nutrition medium only to prevent evaporation. Plates are kept at 37°C, 95% humidity and 5% CO₂ without change of media until 8 days *in vitro* (DIV). Using these culturing conditions, neurons begin to extend processes after a few hours in culture.

### 3.7. Lesion Assays:

For lesioning purpose, 10 µl of the stock solutions - either β-amyloid₂₅₋₃₅, which was pre-aggregated for 72 hours, or L-glutamate - was added to each well except to unlesioned controls (designated as "C" in fig. 2 and 3) at day 8 *in vitro*, to obtain a final concentration of 20 µM β-amyloid₂₅₋₃₅ and a final concentration of 1 mM L-glutamate per well, respectively. The toxic substances remained with the cells for 48 hours. During the lesion cells were maintained in an incubator at 37°C, 95% humidity and 5% CO₂.

### 3.8. Preparation and Administration of Substances

Lyophilized serum S1 was used to prepare stock solutions using distilled water which were further diluted with nutrition medium to achieve the desired concentrations. Serum S1 was added to the wells once at the 1 st day in the appropriate concentrations and remained with the neurons until the end of the experiment. Serum S 1 was tested under identical conditions on two different days, with two equal 96-well microplates on each day (n=8). In every experiment at least 6 particular values were generated for controls (n≥12).

### Example 4. Viability assay

At the end of each experiment, on day 8 (for the low serum cell stress assay) or day 10 (for the β-amyloid and L-glutamate lesion assays), the viability of remaining nerve cells was measured with a colorimetric MTT-reduction assay. This assay is based on the reduction of yellow MTT (3-(4,5- dimethylthiazol-2-yl)-2,5,diphenyl tetrazolium bromide), to dark blue formazan crystals by mitochondrial dehydrogenases (succinate dehydrogenase). Since this reaction is catalysed in living cells only, the assay can be used for the quantification of cell viability. For the determination of cell viability MTT solution was added to each well in a final concentration of 0.5 mg/ml. After 2 h the MTT containing medium was aspirated. Cells were lysed with 3% SDS, formazan crystals dissolved in Isopropanol/HCl. To estimate optical density a plate reader (Anthos HT II) was used at wavelength 570 nm. Neuronal viability is expressed in optical density (OD), as indicated in figures 1, 2 and 3.

### Example 5. Results from the viability assays

The neuroprotective potential of serum S1 was investigated in different *in vitro* assays, namely the 2% low serum cell stress assay, the β-amyloid lesion assay and the acute L-glutamate lesion assay. As described, serum S1 was added to the cultured primary neurons from the first day onwards. Evaluation of possible neuroprotective effects was made 8 (low serum cell stress assay) or 10 days later (lesion assays). Primary neurons grown under the stress conditions mentioned undergo neurodegeneration. Effects of serum S1 are shown in figures 1, 2 and 3, respectively. In the low serum assay (figure 1), addition of serum S1 resulted in a dramatic increase of neuronal viability compared to the untreated control. From a serum concentration of 25 µg/ml onwards neuronal viability increases almost up to 7-fold of the control level. A maximum effect can be achieved with 800 µg/ml. In figure 2, the effects of serum S1 in the β-amyloid lesion assay are shown. It is clearly visible that serum S1 exhibits a clear neuroprotectivity at concentrations greater or equal to 50 µg/ml, whereby the highest effect can be achieved with concentrations from 200 up to 800 µg/ml. Also in the L-glutamate lesion assay serum S1 is able to clearly counteract toxicity of this excitatory amino acid (figure 3). An increase of neuronal viability in the L-glutamate lesion assay can be achieved at concentrations of 50 µg/ml up to 1,600 µg/ml. Since even higher concentrations than 1,600 µg/ml did not lead to neurodegenerative effects, serum S1 appears to be well tolerable in vitro and, thus, likely also *in vivo*.

The general conclusions that can be drawn are:
(1). Evaluating the data obtained, the effective concentrations of serum S1 can be inferred at which a neuroprotective effect is obtained.
(2). Serum S1 showed a clear correlation between the dosage applied and the neuroprotective effects measured.
(3). On the basis of the experimental data, one can assume that even higher concentrations of serum S1 will not lead to neurodegenerative effects. Therefore serum S1 seems to be tolerable *in vitro* and, thus, likely also *in vivo*.

### Example 6. Experiments using hippocampus slices

Experiments using hippocampus slices were designed to learn more on the action of serum S 1 on the brain when exposed directly. In the assays evoked extra-cellular field potentials from the pyramidal cell layer of CA1 are recorded and compared. Specifically, it was tested if serum S 1 has anti-convulsive properties. This was examined by testing the effects which serum S 1 has against pathological changes of the recorded signals induced by various convulsive drugs like pentylenetetrazole and bicuculline.

### Example 7. Preparation of hippocampus slices

Hippocampus slices were obtained from 11 adult male Sprague-Dawlay rats (Charles River Wiga, Sulzbach, Germany). Rats were kept under a reversed day-night cycle for 2 weeks prior start of the experiments, to allow recording of *in-vitro* activity from slices during the active phase of their circadian rhythm (Dimpfel et al., 1994, Dimpfel et al., 1995). Animals were exsanguinated under ether anaesthesia, the brain was removed in total and the hippocampal formation was isolated under microstereoscopic sight. The midsection of the hippocampus was fixed to the table of a vibrating microtome (Rhema Labortechnik, Hofheim, Germany) using a cyanoacrylate adhesive, submerged in chilled bicarbonate-buffered saline (artificial cerebro spinal fluid (ACSF) NaCl: 124 mM; KCl: 5 mM; CaCl₂: 2 mM; MgSO₄: 2 mM; NaHCO₃: 26 mM; and glucose: 10 mM), and cut into slices of 400 µm thickness. All slices were preincubated for at least 1 h in carbogen (30% CO₂ and 70% oxygen)-saturated ACSF (pH 7.4) in a prechamber before use (see also Dimpfel et al., 1991).

During the experiment the slices were held and treated in a special Superfusion chamber (List Electronics, Darmstadt, Germany) at 35°C according to Haas et al., 1979 (see also: Schiff and Somjen, 1985). Four slices were used from one rat per day under one of the test conditions (control or various concentrations of the S1 compound). The preparation was superfused with artificial cerebrospinal fluid (ACSF) at 180-230 ml/h. Electrical stimulation (200 µA constant current pulses of 200 µs pulse width) of the Schaffer Collaterals within the CA2 area and recording of extracellular field potentials from the pyramidal cell layer of CA1 (Dimpfel et al., 1991) as shown in Fig. 4 and Fig. 5 was performed according to conventional electrophysiological methods using the "Labteam" Computer system and "NeuroTool" software package (Medisyst GmbH, Linden, Germany). Measurements were performed at 10 min intervals to avoid potentiation mechanisms. Four stimulations - each 20 seconds apart - were averaged for each time point. After obtaining three stable responses to single stimuli (SS) long term potentiation was induced by applying a theta burst type pattern (TBS; Lynch and Schubert, 1980). The mean amplitude of three signals 20 seconds apart were averaged to give the mean of absolute voltage values (microvolt) +/- standard error of the mean for each experimental condition (single stimulus or theta burst stimulation). Schematic views of the slice preparation are given in Fig. 4 and 5. Epilepsy-like discharges are induced by two convulsive compounds: pentylenetetrazol (which is 6,7,8,9-tetrahydro-5h-tetrazolo(1,5-a)azepine and which is abbreviated PTZ) and bicuculline (see Table 1).

**Table 1**

| | Ch.-B. | from |
|---|---|---|
| Bicuculline | 0110BNQ1 | Bio Trend, 50933 Köln |
| Pentylenetetrazol | BP-391A | Bio Trend, 50933 Köln |

Listing of chemicals used for the experiments.

### Example 8. Influence of Serum S1 on the physiological signal

The effect of serum S1 was tested under physiological conditions. The amplitude of the populations spike produced by recruitment of pyramidal cells was measured during single shock stimulation as well as during theta burst stimulation resulting in long term potentiation (LTP). The presence of serum S 1 in the ACSF medium led to decreases of the population spike amplitude starting with a concentration of 0.25 mg/l. An example is given in Fig. 6 in the presence of 0.75 mg/l of serum S1.

Lowest effective concentration of serum S1 during single stimulus condition was 0.5 mg/l. Strong attenuation was achieved in the presence of 1 mg/l. Using theta burst stimulation suppression was not observed below a concentration of 0.75 mg/l. Further attenuation was observed in the presence of 1 mg/l serum S1. Values in the presence of 0.75 and 1 mg/l of serum S1 were statistically significant (see Table 2).

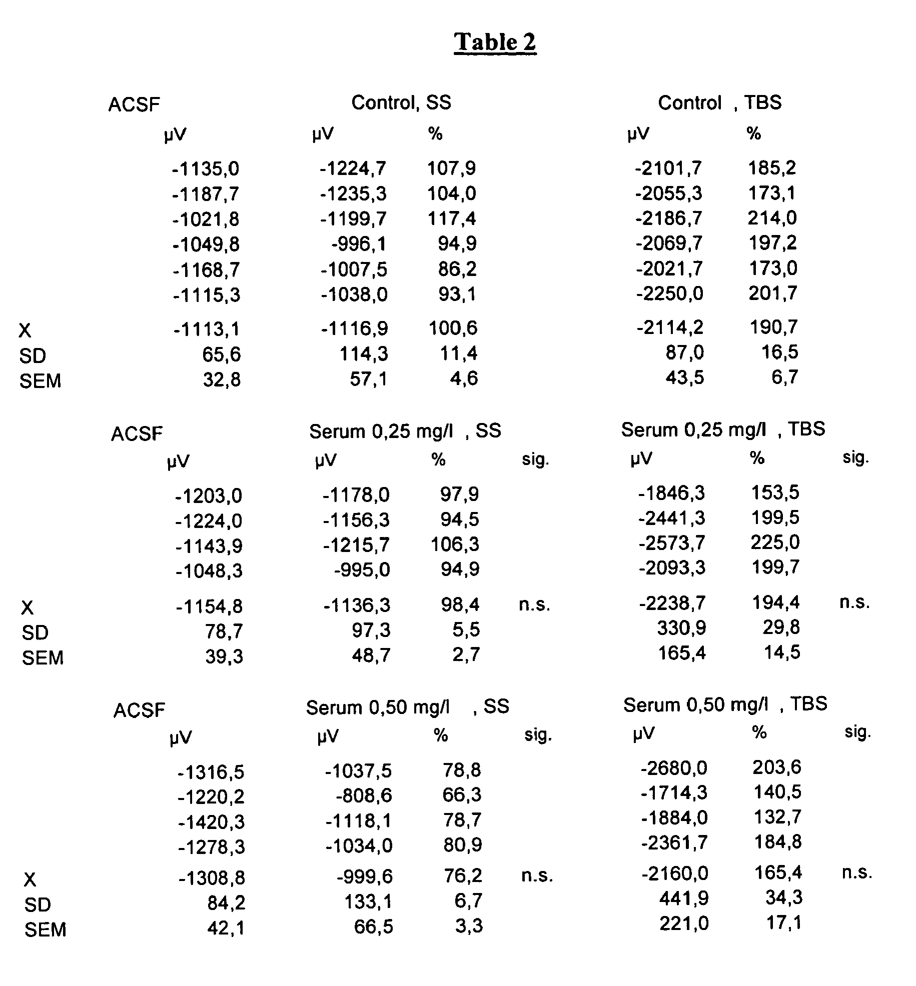

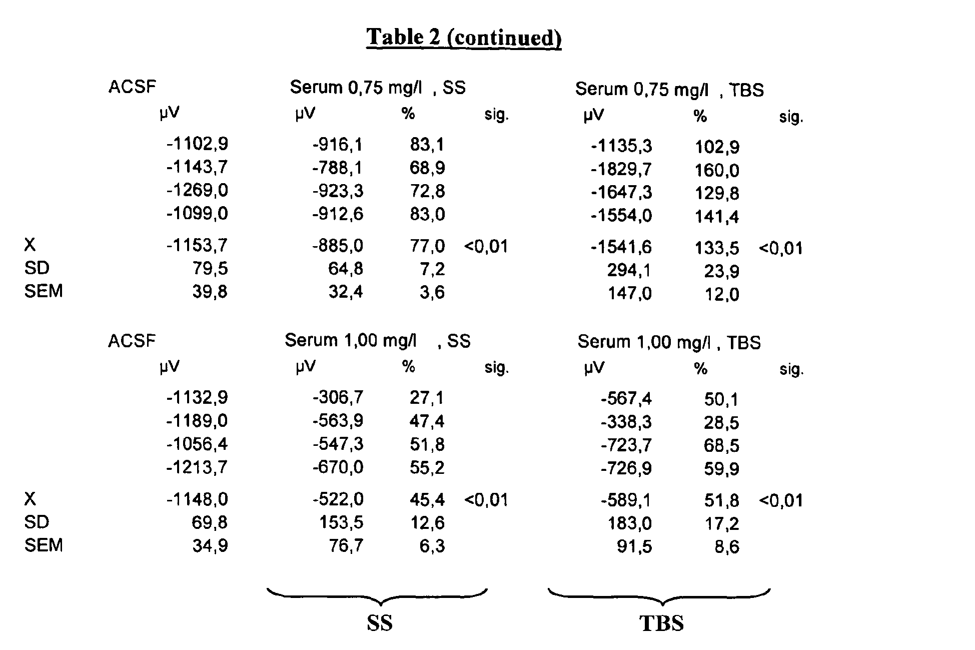

Numerical documentation of results from single slices. Statistical significance from control (values before superfusion with serum S1) is given under the heading "sig.". Absolute values as well as percentage of control are given for each slice. Statistical significances are given under "sig." according to Wilcoxon-Mann-Whitney U-Test. "X" denotes the mean, "SD" refers to the standard deviation, "SEM" is the standard error of the mean, "SS" refers to single stimulation and "TBS" to theta burst stimulation.

Thus, a concentration-dependent effect of serum S 1 could be documented already using single stimuli. Applying theta burst stimulation in order to evoke LTP, a concentration of 0.75 mg/l is needed to change the pyramidal cell response (statistically significant from control with p< 1% error probability). Further attenuation of the amplitude is achieved by superfusion of a concentration of 1 mg/l of serum S1. For example, a reduction to about 50% is obtainable as shown in Fig.7. In summary, S1 serum led to attenuation of the excitability of the synaptic activity pyramidal cells in the hippocampal circuit for both kinds of electrical stimulation.

### Example 9. Antagonism of serum S1 to provocation with convulsive drugs

The physiological condition of the slice can be changed by exposure to chemicals known for their ability to induce epilepsy-like signals after electrical stimulation of the Schaffer Collateral Pathway by single shocks. Two of the most common compounds to change the response of pyramidal cells are pentylenetetrazole and bicuculline. Bicuculline is a competitive antagonist of GABAA receptors. The half-maximal (IC₅₀) effect of bicuculline on GABAA receptors is 3 µM. Pentylenetetrazol (PTZ) is a non-competitive GABA antagonist. Pentylenetetrazole and bicuculline are known to cause convulsions and epileptic discharges within an animal brain.

Examples are given in Fig. 8 for both compounds. Both convulsive compounds increase the amplitude of the signal to a major degree. Only bicuculline induces so-called after discharges (second and third population spike after one single electrical stimulus). At the highest concentration of serum S1 (1 mg/l) pathological increases of the signal amplitude induced by pentylenetetrazole and bicuculline were significantly attenuated. This is documented in Fig. 9. Strongest attenuation through serum S 1 were seen in the presence of bicuculline. Signals nearly returned to control values. Details are given in Table 3.

**Table 3**

| ACSF | | PTZ 50 µM | | PTZ 50µM + Serum 1mg/l | | |
|---|---|---|---|---|---|---|
| | µV | µV | % | µV | % | sig. |
| | -1194,0 | -3035,0 | 254,2 | -2671,0 | 223,7 | |
| | -1169,0 | -2860,7 | 244,7 | -2374,7 | 203,1 | |
| | -1249,0 | -2907,7 | 232,8 | -2223,0 | 178,0 | |
| | -1073,3 | -2616,0 | 243,7 | -2265,3 | 211,1 | |
| X | -1171,3 | -2854,9 | 243,9 | -2383,5 | 204,0 | <0,01 |
| SD | 73,4 | 175,4 | 8,8 | 202,0 | 19,3 | |
| SEM | 36,7 | 87,7 | 4,4 | 101,0 | 9,6 | |

Sensitivity of pentylenetetrazole induced increases of populations spike amplitude in the presence of 1 mg/l of serum S1. A significant effect is observed in the presence of pentylenetetrazole. Statistical significances are given under "sig." according to Wilcoxon-Mann-Whitney U-Test.

In a third experimental series the concentration dependence of the effect of serum S1 on bicuculline-induced pathological increases of the population spike amplitude was measured. As documented in Fig. 10 and Table 4, serum S 1 attenuated the amplitude of the pathological signal induced by 50 pM of bicuculline in a concentration-dependent manner in the range of 0.25 mg/l to 1 mg/l. Induction of a second and third population spike was suppressed. Statistical significance was observed at a concentration of serum S1 of 0.5 mg/l and higher (Table 4).

**Table 4**

| | ACSF | | Bicuculline | 50 µM | Bicuculline 50 µM +Serum 0,25 mg/l | | |
|---|---|---|---|---|---|---|---|
| | | µV | µV | % | µV | % | sig. |
| | | -1014,9 | -2778,0 | 273,7 | -2255,7 | 222,3 | |
| | | -1060,0 | -2524,3 | 238,1 | -2403,3 | 226,7 | |
| | | -1076,1 | -2497,7 | 232,1 | -2435,3 | 226,3 | |
| | | -1025,4 | -2896,0 | 282,4 | -2766,3 | 269,8 | |
| X | | -1044,1 | -2674,0 | 256,6 | -2465,2 | 236,3 | n.s. |
| SD | | 28,8 | 194,5 | 25,2 | 215,5 | 22,4 | |
| SEM | | 14,4 | 97,3 | 12,6 | 107,7 | 11,2 | |

| | ACSF | | Bicuculline | 50 µM | Bicuculline 50 µM +Serum 0,50 mg/l | | |
|---|---|---|---|---|---|---|---|
| | | µV | µV | % | µV | % | sig. |
| | | -1105,5 | -2804,0 | 253,6 | -1969,7 | 178,2 | |
| | | -1056,3 | -2492,0 | 235,9 | -1953,7 | 185,0 | |
| | | -1046,5 | -2729,0 | 260,8 | -2148,7 | 205,3 | |
| | | -1211,0 | -2958,7 | 244,3 | -1805,3 | 149,1 | |
| X | | -1104,8 | -2745,9 | 248,7 | -1969,4 | 179,4 | <0.01 |
| SD | | 75,3 | 194,4 | 10,9 | 140,6 | 23,3 | |
| SEM | | 37,7 | 97,2 | 5,4 | 70,3 | 11,6 | |

| | ACSF | | Bicuculline | 50 µM | Bicuculline 50 µM +Serum 0,75 mg/l | | |
|---|---|---|---|---|---|---|---|
| | | µV | µV | % | µV | % | sig. |
| | | -1167,3 | -2604,0 | 223,1 | -1407,7 | 120,6 | |
| | | -1052,3 | -2678,7 | 254,6 | -1640,3 | 155,9 | |
| | | -1124,7 | -2585,7 | 229,9 | -1531,0 | 136,1 | |
| | | -1181,4 | -2869,7 | 242,9 | -1490,7 | 126,1 | |
| X | | -1131,4 | -2684,5 | 237,6 | -1517,4 | 134,7 | <0.01 |
| SD | | 58,0 | 129,8 | 14,0 | 96,7 | 15,5 | |
| SEM | | 29,0 | 64,9 | 7,0 | 48,3 | 7,8 | |

| | ACSF | | Bicuculline | 50 µM | Bicuculline 50 µM +Serum 1,00 mg/l | | |
|---|---|---|---|---|---|---|---|
| | | µV | µV | % | µV | % | sig. |
| | | -996,5 | -2580,0 | 258,9 | -1162,3 | 116,6 | |
| | | -1178,0 | -2452,7 | 208,2 | -1214,0 | 103,1 | |
| | | -1102,0 | -2942,3 | 267,0 | -1239,0 | 112,4 | |
| | | -1162,0 | -3070,7 | 264,3 | -1289,7 | 111,0 | |
| X | | -1109,6 | -2761,4 | 249,6 | -1226,3 | 110,8 | <0.01 |
| SD | | 82,2 | 292,5 | 27,8 | 53,0 | 5,6 | |
| SEM | | 41,1 | 146,2 | 13,9 | 26,5 | 2,8 | |

Attenuation of the pathological signal, induced by 50 mM of bicuculline, in the presence of different concentrations of serum S1. Statistical significances are given under "sig." according to Wilcoxon-Mann-Whitney U-Test. "X" denotes the mean, "SD" refers to the standard deviation and "SEM" is the standard error of the mean.

In summary, serum S1 selectively interferes with pathological changes induced by chemicals known to induce convulsions in animals. Pentylenetetrazole induced discharges were moderately attenuated and full efficacy was observed against bicuculline induced epilepsy-like discharges of hippocampal pyramidal cells in a concentration range between 0.25 mg/l and 1 mg/l.

### Example 10. Summary of the results from experiments using hippocampus slices

Rat hippocampus slice preparation were used to detect pharmacological effects of serum S1 *in vitro*. Electrical stimulation of the Schaffer Collaterals results in activation of pyramidal cells by means of a glutamatergic synapse. The physiological signal was attenuated by serum S1 in a concentration dependent manner in the range of 0.5 to 1 mg/l during single shock stimulation and in the range of 0.75 to 1 mg/l during theta burst stimulation. Induction of pathological increases of the population spike amplitude by convulsive drugs was attenuated selectively. Attenuation was seen in the presence of 50 µM pentylenetetrazole and full concentration dependent attenuation was observed when using 50 µM of bicuculline. This attenuation already was visible at a concentration of 0.25 mg/l of serum S1. Serum S1 mediated attenuation was observed independent of whether single stimulation or theta burst stimulation was applied. This feature is shared by drugs functioning as GABA receptor agonists.

### Example 11 - Electropharmacogram experiments

Drugs exert their action within the organism by interacting with biochemically defined targets. With respect to the central nervous system, neurotransmitter receptors represent main targets of drugs. Interaction of drugs with these targets induces a signalling cascade which finally ends up with the control of ion channel conductances. Since the electrical activity of single neurons depends on the set of momentarily active ion channels, communication between neurons is governed by these processes. Thus, field potentials carry the information of larger local networks of electrically active neurons and reflect the effects that drugs have on their targets.

Frequency analysis of the field potentials in the presence of drugs leads to the so-called electropharmacogram which has been widely used in the past to characterize drug actions on rat (Dimpfel, 2007) and human brains. Interpretation of the results was performed with respect to neurotransmitter activity as well as aiming at possible clinical indications in humans. The relationship between delta waves and cholinergic transmission has been reported for the first time by Dimpfel (2005). Theta waves have been recognized as being influenced by drugs acting at the norepinephrine alpha2 receptor (Dimpfel and Schober, 2001). Presynaptic interaction with this receptor leads to drowsiness and sleep and changes of theta waves have been used as part of a formula describing depth of sleep in humans (Dimpfel et al., 1998).

EEG signals were recorded from frontal cortex, hippocampus, striatum and reticular formation of freely moving rats from inside a totally copper shielded room. Signals were wirelessly transmitted by a radio-telemetric system (Rhema Labortechnik, Hofheim, Germany, using 40 Megahertz as carrier frequency) and were amplified and processed as described earlier to give power spectra of 0.25 Hz resolution (Dimpfel et al. 1988; Dimpfel et al. 1989; Dimpfel, 2003). In short, after automatic artefact filtering signals were collected in sweeps of 4 s duration and Fast Fourier transformed using a Hanning window. Sampling frequency was 512 Hz. Four values were averaged to give a final sampling frequency of 128 Hz, well above the Nyquist frequency. The resulting electrical power spectra were divided into 6 specially defined frequency ranges (delta: 0.8 - 4.5 Hz; theta: 4.75 - 6.75 Hz; alpha1: 7.00 - 9.50 Hz; alpha2: 9.75 - 12.50 Hz; beta1: 12.75 - 18.50 Hz; beta2: 18.75 - 35.00 Hz). Spectra were averaged in steps of 3 minutes each and displayed on-line. In an off-line procedure spectra were averaged to give longer periods for further analysis and data presentation.

For the following experiments, adult Fisher 344 rats (8 month of age and day - night converted, weight about 400 g, provided by Charles River Laboratories, D-97633, Sulzfeld) were used. Into the brain of each rat 4 bipolar concentric steel electrodes were implanted using a stereotactic surgical procedure and Ketamine-induced anaesthesia. All four electrodes were placed 3 mm lateral within the left hemisphere. Dorsoventral coordinates were 4, 6, 4.2 and 8 mm and anterior coordinates were 3.7, 9.7, 5.7 and 12.2 mm for frontal cortex, striatum, hippocampus, and reticular formation, respectively (according to the atlas of Paxinos and Watson, 1982). A preconstructed base plate carrying 4 bipolar stainless steel semi-micro electrodes (neurological electrodes "SNF 100" from Rhodes Medical Instruments, Inc., Summerland, CA 93067, USA) and a 5-pin-plug was fixed to the skull by dental cement interacting with 3 steel screws placed on distance into the bone. The distant recording spot of the electrode was the active electrode whereas the proximal spots of the four electrodes were connected to each other to give a reference. The base plate was carrying a plug to receive later on the signal transmitter (weight: 5.2 g including battery, 26x12x6 mm of size). Animals were given two weeks for recovery from surgery.

After surgery all animals were randomly allocated to the treatment group, such that the treatment groups were evenly distributed throughout the caging system. A crossover design with at least one week of drug holidays in between the administrations was used. Controls consisted of i.p. administration of 1 ml/kg of a Saline solution. Active treatment consisted in administration of three dosages of Serum S1. After a pre-drug period of 45 minutes for baseline recording, drug effects were observed continuously for 300 minutes subdivided into 15 min periods, after a lag time of 5 minutes for calming of animals after intraperitoneal administration. Changes of electrical power (µV2/W) are expressed as % of the 45 min. absolute pre-drug spectral power values within each frequency band. Data were averaged from all 8 animals. Serum S1G (50 mg/kg) was tested only on 7/8 animals (one animal of the group had lost the implant). Data are expressed as mean values +- S.E.M. Statistics were calculated by means of the Wilcoxon-Mann-Whitney U-test.

During the recording rats were not restricted and could move freely but did not have food available (chewing would have produced too many artefacts). The animals were allowed to acclimatise for at least 4 weeks before the study started. There was automatic control of light cycle, temperature and humidity. Light hours were 18h in the evening - 6h in the morning. Daily monitoring indicated that temperature and humidity remained within the target ranges of 22°C ± 2°C and 44% ± 5% respectively. Cages, bedding, and water bottles were changed at regular intervals, i.e. every 2-3 days. Standard Diet (Nohrlin H10, Altromin, D-32791 Lage, Germany) was available to the animals ad libidum. The animals had access to domestic quality mains water ad libidum.

### Example 11.1 Effect of Saline on the Electropharmacogram

Intraperitoneal administration of physiological saline did not result in any significant changes of spectral power in comparison to the 45 min lasting pre-drug reference time (set to 100%). A time course is depicted in Fig. 11 for the whole recording period of 5 hours after administration of 1 ml/kg of the saline.

### Example 11.2 Effect of serum S1 on the Electropharmacogram

The biologically active blood serum of the invention (also called "serum S1" herein) was prepared fresh for each experimental day and injected intraperitoneally after 45 minutes of pre-drug baseline recording.

Administration of a middle dosage of 100 mg/kg of serum S 1 resulted in some statistically significant decreases in theta and alpha1 power during the first hour of recording after dosing. Maximal effects were seen during the second hour. During this time decreases in spectral power could be documented within all brain areas. These changes were statistically significant from control with respect to all brain areas. Strongest effects were recognized with the frontal cortex and hippocampus, clearly less in the striatum and reticular formation (Fig. 12). After this effects ceased with time. The strong effect on the theta waves may be an indication that the serum of the invention can be used to treat insomnia.

The effects of the biologically active serum according to the invention was further dose dependent as seen in fig. 13.

### Example 11.3 Pharmaceutically active compounds having related effects on the electropharmacogram as serum S1

A database comprising electropharmacogram data of 200 pharmaceutically active compounds was searched to reveal compounds having a similar electropharmacogram as is obtainable by administering serum S 1. Up to now no drug could be identified having an identical pattern of changes based on the proportional changes of single frequency ranges, indicating that the biologically active blood serum of the invention exhibits a unique mode of action, perhaps modulating next to the activity of GABA receptors also the activity of further receptors. However, some compounds from the database showed partially related wave patterns as obtained when administering serum S1. These compounds are shown in fig. 14 and comprise tacrine (fig. 14) and donepezil (not shown) which are acetylcholinesterase inhibitors that can be used to treat cognitive impairment associated with multiple sclerosis and schizophrenia, providing further evidence that the biologically active blood serum of the invention can be used for the prevention or therapy of a disorder characterized in a reduced function of a GABA receptor, in particular for the prevention or therapy of a substance-abuse related disorder, multiple sclerosis, an aging related neuropathy, pain, schizophrenia, Huntington's disease, insomnia, asthma and a movement disorder.

### References:

Dimpfel W (2007), "Characterization of atypical antipsychotic drugs by a late decrease of striatal alpha1 spectral power in the electropharmacogram of freely moving rats"; Br J Pharmacol 152: 438-48
Dimpfel W, Spüler M, Dalhoff A, Hoffmann W, Schlüter G (1991), "Hippocampal activity in the presence of quinolones and fenbufen in-vitro"; Antimicrobial Agents and Chemotherapy; June: 1142-TL46.
Dimpfel W (2003), "Preclinical data base of pharmaco-specific rat EEG fingerprints (Tele-Stereo-EEG)"; Eur J Med Res 8: 199-207.
Dimpfel W, Spüler M, Borbe HO (1988), "Monitoring of the effects of antidepressant drugs in the freely moving rat by radioelectroecephalography (Tele-Stereo-EEG)", Neurobiology 19: 116-120.
Dimpfel W, Spüler M, Nichols DE (1989), "Hallucinogenic and stimulatory amphetamine derivatives: fingerprinting DOM, DOI, DOB, MDMA and MBDB by spectral analysis of brain field potentials in the freely moving rat (Tele-Stereo-EEG)". Psychopharmacology 98:297-303.
Dimpfel (1998), "Validation of an EEG-derived spectal frequency index (SFx) for continuous monitoring of sleep depth in humans"; Eur J Med Res, 3: 453-60.
Dimpfel W (2005), "Pharmacological modulation of cholinergic brain activity and its reflection in special EEG frequency ranges from various brain areas in the freely moving rat (Tele-Stereo-EEG)"; Eur. Neuropsychopharmacol; 15: 673-682.
Dimpfel W, Schober F (2001) "Norepinephrine, EEG theta waves and sedation in the rat".
Dimpfel W, Dalhoff B, Hofmann W, Schlüter G (1994), "Electrically evoked potentials in the rat hippocampus slice in the presence of aminophylline alone and in combination with quinolones"; European Neuropsychopharmacology 4 : 15 1 - 156.
Dimpfel W. (1995), "Effects of Memantine on Synaptic Transmission in the Hippocampus in vitro"; Arzneim.-Forsch./DrugRes.45 (I), 1, 1 - 5.
Dimpfel W (1996), "Effect of thioctic acid on pyramidal cell responses in the rat hippocampus in vitro"; Eur J Med Res. 1: 523-527.
Haas HL, Schaerer B, Vosmansky M (1979), "A simple perfusion chamber for the study of nervous tissue slices in vitro"; J Neurosci Methods 1: 323-5.
Lynch G, Schubert P (1980), The use of in-vitro brain slices for multidisciplinary studies of synaptic function. Ann Rev Neurosci, 3 : 1-22.
Schiff SJ, Somjen GG (1985), "The Effects of Temperature on Synaptic Transmission in Hippocampal Tissue Slices", Brain Research 345: 279-284.

## Claims

1. Use of a biologically active blood serum producible according to a method comprising the steps of:
a) electrostimulation of a non-human animal
b) withdrawal of blood from said animal,
c) isolation of serum from said blood, and
d) gamma irradiation of said serum
for the manufacture of a medicament for the prevention or therapy of a disorder **characterized by** a reduced function of a GABA receptor.

2. Use according to claim 1, wherein the disorder is selected from a group consisting of a substance-abuse related disorder, multiple sclerosis, an aging related neuropathy, pain, schizophrenia, Huntington's disease, insomnia, asthma and a movement disorder.

3. A biologically active blood serum producible according to a method comprising the steps of:
a) electrostimulation of a non-human animal
b) withdrawal of blood from said animal,
c) isolation of serum from said blood, and
d) gamma irradiation of said serum
for the prevention or therapy of a disorder **characterized in** a reduced function of a GABA receptor.

4. Biologically active blood serum according to claim 3, wherein the disorder is selected from a group consisting of a substance-abuse related disorder, multiple sclerosis, an aging related neuropathy, pain, schizophrenia, Huntington's disease, insomnia, asthma, and a movement disorder.

5. A pharmaceutical composition comprising:
(i) a biologically active blood serum producible according to a method comprising the steps of:
(a) electrostimulation of a non-human animal
(b) withdrawal of blood from said animal,
(c) isolation of serum from said blood, and
(d) gamma irradiation of said serum
and
(ii) at least one pharmaceutically active ingredient selected from the group consisting of an analgesic, a GABA receptor agonist, an acetylcholinesterase inhibitor, an NMDA receptor antagonist, a dopamine receptor blocker, a corticosteroid, creatine, CoQ10, minocycline, a serotonin reuptake inhibitor (SSRI), a dopamine antagonist, a dopa decarboxylase inhibitor, L-dopa, a catechol-O-methyl transferase inhibitor, a monoamine oxidase-B (MAO-B) inhibitor, an antiglutamatergic agent, CEP 1347, CTCT346, a lazaroid, erythropoietin, methylprednisolone a dopamine receptor and derivatives thereof.

6. The pharmaceutical composition according to claim 5, wherein the acetylcholinesterase inhibitor is selected from the group consisting of metrifonate, carbamate, physostigmine, neostigmine, pyridostigmine, ambenonium, demarcarium, rivastigmine, a phenanthrine derivative, galantamine, a piperidine, donepezil, tacrine, edrophonium and phenothiazine.

7. The pharmaceutical composition according to claim 5, wherein the NMDA receptor antagonist is selected from the group consisting of amantadine, dextromethorphan, dextrorphan, ibogaine, ketamine, nitrous oxide, phencyclidine, riluzole, tiletamine, ethanol , dizocilpine, aptiganel, memantine, remacimide, 7-chlorokynurenate, 5,7-dichlorokynurenic, kynurenic acid, 2-amino-7-phosphonoheptanoic acid, r-2-amino-5-phosphonopentanoate and 3-[(R)-2-carboxypiperazin-4-yl]-prop-2-enyl-1-phosphonic acid).

8. The pharmaceutical composition according to claim 5, wherein the dopamine antagonist is selected from the group consisting of clozapine, risperidone, olanzapine, quetiapine, ziprasidone, aripiprazole, metoclopramide, droperidol, domperidone, chlorpromazine and amoxapine.

9. The pharmaceutical composition according to claim 5, wherein the GABA receptor agonist is selected from the group consisting of β-carbolin-3-carboxamide, FG-7142, etomidat, propofol, gamma-hydroxybutyric acid, benzodiazepine, zolpidem, zopiclone, zaleplon, methaqualone, baclofen, muscimol, progabide, tiagabine, 4-aminobutanoic acid (GABA); 3-aminopropyl)methylphosphinic acid; 4-amino-3-phenylbutanoic acid; 4-amino-3-hydroxybutanoic acid; 4-amino-3-(4-chlorophenyl)-3-hydroxyphenylbutanoic acid; 4-amino-3-(thien-2-yl)butanoic acid; 4-amino-3-(5-chlorothien-2-yl)butanoic acid; 4-amino-3-(5-bromothien-2-yl)butanoic acid; 4-amino-3-(5-methylthien-2-yl)butanoic acid; 4-amino-3-(2-imidazolyl)butanoic acid; 4-guanidino-3-(4-chlorophenyl)butanoic acid; 3-amino-2-(4-chlorophenyl)-1-nitropropane; (3-aminopropyl)phosphonous acid; (4-aminobut-2-yl)phosphonous acid; (3-amino-2-methylpropyl)phosphonous acid; (3-aminobutyl)phosphonous acid; (3-amino-2-(4-chlorophenyl)propyl)phosphonous acid; (3-amino-2-(4-chlorophenyl)-2-hydroxypropyl)phosphonous acid; (3-amino-2-(4-fluorophenyl)propyl)phosphonous acid; (3-amino-2-phenylpropyl)phosphonous acid; (3-amino-2-hydroxypropyl)phosphonous acid; (E)-(3-aminopropen-1-yl)phosphonous acid; (3-amino-2-cyclohexylpropyl)phosphonous acid; (3-amino-2-benzylpropyl)phosphonous acid; [3-amino-2-(4-methylphenyl)propyl]phosphonous acid; [3-amino-2-(4-trifluoromethylphenyl)propyl]phosphonous acid; [3-amino-2-(4-methoxyphenyl)propyl]phosphonous acid; [3-amino-2-(4-chlorophenyl)-2-hydroxypropyl]phosphonous acid; (3-amino propyl)methylphosphinic acid; (3-amino-2-hydroxypropyl)methylphosphinic acid; (3-aminopropyl)(difluoromethyl)phosphinic acid; (4-aminobut-2-yl)methylphosphinic acid; (3-amino-1-hydroxypropyl)methylphosphinic acid; (3-amino-2-hydroxypropyl)(difluoromethyl)phosphinic acid; (E)-(3-aminopropen-1-yl)methylphosphinic acid; (3-amino-2-oxo-propyl)methyl phosphinic acid; (3-aminopropyl)hydroxymethylphosphinic acid; (5-aminopent-3-yl)methylphosphinic acid; (4-amino-1,1,1-trifluorobut-2-yl)methylphosphinic acid; (3-amino-2-(4-chlorophenyl)propyl)sulfinic acid; 3-aminopropylsulfinic acid and 1-(aminomethyl)cyclohexaneacetic acid.

10. The pharmaceutical composition according to claim 5, wherein the analgesic is selected from the group consisting of acetaminophen (paracetamol), aspirin, opioid analgesics, such as morphine, codeine, dihydrocodeine, diacetylmorphine, bydrocodone, hydromorphone,levorphanol, oxymorphone, alfentanil, buprenorphine, butorphanol, fentanyl, sufentanyl, meperidine, methadone, nalbuphine, propoxyphene, pentazocine and pharmaceutically acceptable salts thereof.

11. The biologically active blood serum according to claim 3 or 4 and the pharmaceutical composition according to any of claims 5 to 10, wherein said biologically active blood serum and/or said pharmaceutical composition further comprises one or more pharmaceutically acceptable diluents; carriers; excipients, including fillers, binders, lubricants, glidants, disintegrants, adsorbents; and/or preservatives.

12. The biologically active blood serum according to claim 3 or 4 and the pharmaceutical composition according to any of claims 5 to 10, wherein said biologically active blood serum and/or said pharmaceutical composition is formulated as a syrup, an infusion or injection solution, a tablet, a capsule, a capslet, lozenge, a liposome, a suppository, a plaster, a band-aid, a retard capsule, a powder, or a slow release formulation. Preferably the diluent is water, a buffer, a buffered salt solution or a salt solution and the carrier preferably is selected from the group consisting of cocoa butter and vitebesole.

13. The biologically active blood serum according to claim 3 or 4 and the pharmaceutical composition according to any of claims 5 to 10, wherein said biologically active blood serum and/or said pharmaceutical composition is administered to a subject in need of treatment of or prophylaxis for a disorder **characterized in** a reduced function of a GABA receptor in an amount ranging from 1 to 500 mg/kg body weight, preferably ranging from 10 to 200 mg/kg body weight.
